# EUROPEAN PATENT SPECIFICATION

(11) **EP 2 307 399 B1**
(45) Date of publication and mention of the grant of the patent: **18.05.2016**
(21) Application number: 08789975.3
(22) Date of filing: 09.06.2008
(51) Int. Cl.: C07D 333/56, B01D 9/00

(54) **PROCESS FOR CONTROLLING THE GROWTH OF A RALOXIFENE HYDROCHLORIDE CRYSTAL**
VERFAHREN ZUR STEUERUNG DES RALOXIFENE HYDROCHLORID KRISTALLWACHSTUMS
PROCÉDÉ DE RÉGULATION DE LA CROISSANCE D'UN CRISTAL DE CHLORHYDRATE DE RALOXIFÈNE

(43) Date of publication of application: 13.04.2011
(73) Proprietor: ERREGIERRE S.p.A., 24060 San Paolo D'Argon (Bergamo) (IT)
(72) Inventor: FERRARI, Massimo, I-24069 Cenate Sotto (IT); ALBERELLI, Manuel, I-24060 Grone (IT); AMBROSINI, Alberto, I-24040 Osio Sopra (IT)
(74) Representative: Cattaneo, Elisabetta
(86) International application number: PCT/IT2008/000380
(87) International publication number: WO 2009/150669

(56) References cited:
- EP-A- 0 062 503
- WO-A-2005/003116
- W.K. BURTON, N. CABRERA AND F.C. FRANK: "The Growth of Crystals and the Equilibrium Structure of their Surfaces" PHILOSOPHICAL TRANSACTIONS OF THE ROYAL SOCIETY A, vol. 243, 1951, - 1951 pages 299-358, XP002522524 London
- KIRK-OTHMER: "encyclopedia of chemical technology volume 7 crystallisation" 1993, WILEY-INTERSCIENCE PUBLICATION , NEW YORK , XP002522525 page 683 - page 729
- Jörg Worlitschek ET AL: "Model-Based Optimization of Particle Size Distribution in Batch-Cooling Crystallization of Paracetamol", Crystal Growth & Design, vol. 4, no. 5, 1 September 2004 (2004-09-01), pages 891-903, XP055157924, ISSN: 1528-7483, DOI: 10.1021/cg034179b

## Description

### FIELD OF THE INVENTION

The present invention relates to a process for controlling the growth of a raloxifene hydrochloride crystal.

### STATE OF THE ART

Raloxifene and in particular the relative hydrochloride salt, characterized by the following formula (I): 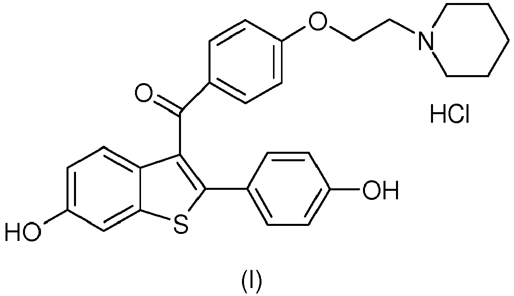 is an active principle used in the treatment of osteoporosis and was described for the first time in European patent application EP62503. According to said patent, the active principle is obtained in the form of a hydrochloride at low yields by a process that comprises a purification step by column chromatography to remove certain by-products.

With the aim of improving the yield and purity of the product, in the international patent application WO2005/003116 a multi-step process for obtaining raloxifene hydrochloride is described, which is characterized by a step of hydrolyzing 6-acetoxy-2-(4-acetoxyphenyl)3-[4-(2-piperidinoethoxy)benzoyl]-benzo[b]thiophene with alkaline hydroxide in an alcoholic solvent and subsequent acidification of the obtained product with concentrated hydrochloric acid. According to that described in the international patent application, crude raloxifene hydrochloride is obtained with a purity equal to about 98% and a yield of about 65-70%.

Specifically, example 3 of the international application describes the obtaining of crystallized raloxifene hydrochloride with a purity of 99%, which can then be further purified, as described in example 4, in methanol and water in the presence of 37% hydrochloric acid to increase its purity. The product obtained in this manner has an HPLC purity higher than 99.8% with particle size characteristics such that D(0.9) is ≤ 100 µm and D(0.5) is ≥ 40 µm. Following a further sieving step, the product presented the following particle size characteristics: D(0.9) comprised between 50 and 65 µm and D[4.3] ≥ 20 µm.

For the purpose of marketing the raloxifene hydrochloride product, it is felt the need that the product can have different product particle size distributions, and not necessarily with particle size characteristics of D(0.9) ≤ 100 µm and D(0.5) ≥ 40 µm.

The object of the present invention is therefore to provide control of granule growth, i.e. the size of single raloxifene hydrochloride crystals, during implementation of the process itself, so as to obtain a precise and desired final raloxifene hydrochloride crystal size.

### SUMMARY

The aforementioned object was achieved by means of a process for controlling the growth of a raloxifene hydrochloride crystal having particle size distribution D(0.9) ≤ 100µm and D(0.5) ≥ 35µm which comprises the steps of:
a) heating under reflux at a temperature in the range of 65 to 67°C a mass comprising crystalline raloxifene hydrochloride, methanol and water for a time of about 10 minutes,
b) cooling the mass to 30-35ºC to interrupt crystal growth,
c) checking the crystal particle size distribution,
wherein steps a), b) and c) are repeated for a number of cycles n, wherein n is from 2 to 5, until said particle size distribution is obtained and wherein once the desired crystal size has been achieved, the cooled mass is distilled under vacuum to remove part of the solvent.

### DETAILED DESCRIPTION

The invention therefore relates to a process for controlling raloxifene hydrochloride crystal growth, i.e. for controlling the size of the raloxifene hydrochloride crystal, as claimed in appended claim 1.

The crystalline raloxifene hydrochloride compound of step a) is preferably obtained as described in the international patent application WO2005/003116, i.e. by crystallization of crude raloxifene hydrochloride in alcoholic solvent, preferably from a mass comprising methanol-water in the presence of HCl. More preferably, according to the invention, the weight ratio of water to methanol in the mass for obtaining the crystallized raloxifene of step a) from crude raloxifene hydrochloride is about 1:12.

The crude raloxifene hydrochloride from which the crystalline raloxifene hydrochloride of step a) is obtained, is preferably obtained by the process described in international patent application WO2005/003116, which is included herein for reference, in particular for examples 1 and 2 which lead to the obtainment of crude raloxifene hydrochloride.

The weight ratio of water to methanol in step a) is preferably about 1: 90 and the reflux temperature is in the range from 65 to 67ºC.

Without wishing to be bound to any theory, the inventors have found that the maintenance time at reflux of 10 minutes is a sufficient time and necessary for the purpose of achieving an effective control of raloxifene hydrochloride crystal size during implementation of the process for preparing crystalline raloxifene hydrochloride.

In step b) of cooling to 30-35ºC, crystal growth is interrupted.

The cooling step b) is followed by the crystal size checking step c). Said step preferably comprises sampling of the reaction mass, filtering and drying the sample followed by checking the particle size distribution by a laser technique on the sample. Following to step c), the size of the sample will be representative of the crystal sizes present in the reactor.

According to the process of the invention, therefore, the mass comprising crystalline raloxifene hydrochloride, methanol and water of step a) is heated under reflux at a temperature in the range of 65 to 67ºC for about 10 minutes, then cooled to 30-35ºC in step b), after which the size achieved by the crystal is checked. The number of cycles n, will therefore vary from 2 to 5, according to the desired crystal size. The number of cycles will be greater the greater the required crystal size.

Once the desired crystal size has been achieved through sampling of the crystal and a suitable number of cycles, the cooled mass is distilled under vacuum to remove a part of the solvent without further increasing particle size of the crystalline product.

The invention will now be described by referring to some examples of the process of the invention for obtaining the desired particle size distribution of the raloxifene hydrochloride crystal by way of non-limiting illustration.

### Example 1

### Preparation of crystallized raloxifene hydrochloride

228 kg of water, crude raloxifene hydrochloride corresponding to 152 kg of dry product (prepared as described in example 2 of international patent application WO2005/003116) and 2711 kg of methanol were loaded into a reactor.

The mass was heated to 62-68ºC until completely dissolved, then treated with a suspension of 6.4 kg of decolorizing carbon in 25.3 kg of methanol.

After having removed the carbon by filtration, 1693 kg of methanol were distilled off.

The residue was cooled to 30°-35ºC and 2.54 kg of 37% hydrochloric acid were added until pH ≤ 2.0 was reached.

The mass was stirred at 30-35ºC for at least 2 hours then centrifuged, washing with 152 kg of cold methanol.

The wet product was then used (after determining loss on drying) in the next step for obtaining raloxifene hydrochloride of the desired particle size.

Based on the loss on drying, the quantity of obtained crude product was 109 kg.

### Example 2

### Preparation of raloxifene hydrochloride with a particle size distribution equal to D(0.5)= 41.2 µm and D(0.9)= 93.0 µm from the crystallized raloxifene of example 1 with D(0.5)= 15.7 µm and D(0.9)= 45.3 µm

22 kg of water, crystallized raloxifene hydrochloride obtained from example 1 with D(0.5)= 15.7 µm and D(0.9)= 45.3 µm corresponding to 109 kg of dry product, and 1962 kg of methanol were loaded into a reactor.

The mass was heated under reflux for about 10 minutes, then cooled to 25 -35ºC and the particle size distribution was checked on a small sample withdrawn from the reactor.

The cycle which comprised heating under reflux for about 10 minutes, cooling to 25 - 35ºC and checking particle size by withdrawing a sample, was repeated four times to obtain the particle size distribution values shown in the following Table 1:

**Table 1**

| Example 1 | D(0.1) µm | D(0.5) µm | D(0.9) µm |
|---|---|---|---|
| Crystallized raloxifene hydrochloride (starting product) | 4.3 | 15.7 | 45.3 |
| 1st cycle (first check during the process) | 4.9 | 20.6 | 55.7 |
| 2nd cycle (second check during the process) | 6.1 | 25.8 | 63.0 |
| 3rd cycle (third check during the process) | 8.2 | 32.6 | 76.5 |
| 4th cycle (fourth check during the process) | 13.1 | 42.2 | 92.1 |

When growth was complete, 982 kg of methanol were distilled off under vacuum. The residue was cooled to 30-35ºC and 2.18 kg of 37% hydrochloric acid were added until pH ≤ 2.0 was reached.

The mass was stirred at 30-35ºC for at least 2 hours, then the suspension was centrifuged, washing with 109 kg of cold methanol.

The wet product was dried at 70-80ºC.

The dry product was passed through a granulator (moderate grinding conducted with the aim of homogenising the product). 97 kg of dry product with D(0.1)= 10.3 µm D(0.5) = 41.2 µm and D(0.9)= 93.0 µm were obtained.

### Example 3

### Preparation of raloxifene hydrochloride with a particle size distribution of D(0.5)= 36.6 µm and D(0.9)= 86.7 µm from the crystallized raloxifene of example 1 with D(0.5)= 18.4 µm and D(0.9)= 53.2 µm

By using the same ingredients and amounts and following the procedure of example 2, but starting from the crystallized raloxifene of example 1 with D(0.5)= 18.4 µm and D(0.9)= 53.2 µm, the steps of heating under reflux, cooling and checking were carried out three times to obtain the particle sizes indicated in the following Table 2.

**Table 2**

| Example 3 | D(0.1) µm | D(0.5) µm | D(0.9) µm |
|---|---|---|---|
| Crystallized raloxifene hydrochloride (starting product) | 4.5 | 18.4 | 53.2 |
| 1st cycle (first check during the process) | 5.0 | 21.8 | 61.3 |
| 2nd cycle (second check during the process) | 7.5 | 30.2 | 77.0 |
| 3rd cycle (third check during the process) | 9.0 | 35.5 | 89.8 |

The product leaving the third treatment cycle was then dried and granulated as in example 2 to obtain raloxifene hydrochloride with D(0.1)= 10.0 µm D(0.5) = 36.6 µm and D(0.9)= 86.7µm.

### Example 4

### Preparation of raloxifene hydrochloride with a particle size distribution equal to D(0.5)= 38.4 µm and D(0.9)= 87.7 µm from the crystallized raloxifene of example 1 with D(0.5)= 14.7 µm and D(0.9)= 39.5 µm

By using the same ingredients and amounts and following the procedure of example 2, but starting from the crystallized raloxifene of example 1 with D(0.5)= 14.7 µm and D(0.9)= 39.5 µm the steps of heating under reflux, cooling and checking were carried out five times to obtain the particle sizes indicated in the following Table 3.

**Table 3**

| Example 4 | **D(0.1) µm** | **D(0.5) µm** | **D(0.9) µm** |
|---|---|---|---|
| Crystallized raloxifene hydrochloride (starting product) | 3.8 | 14.7 | 39.5 |
| 1st cycle (first check during the process) | 3.6 | 16.3 | 41.2 |
| 2nd cycle (second check during the process) | 4.5 | 20.7 | 50.2 |
| 3rd cycle (third check during the process) | 7.5 | 30.3 | 68.2 |
| 4th cycle (fourth check during the process) | 8.5 | 35.1 | 78.5 |
| 5th cycle (fifth check during the process) | 9.0 | 38.4 | 87.3 |

The product leaving the fifth treatment cycle was then dried and granulated as in example 2 to obtain raloxifene hydrochloride with D(0.1)= 8.5µm D(0.5) = 38.4 µm and D(0.9)= 87.7µm.

### Example 5

### Preparation of raloxifene hydrochloride with particle size distribution equal to D(0.5)= 37.4 µm and D(0.9)= 94.1 µm from the crystallized raloxifene of example 1 with D(0.5)= 22.8 µm and D(0.9)= 71.6 µm

By using the same ingredients and amounts and following the procedure of example 2, but starting from the crystallized raloxifene of example 1 with D(0.5)= 14.7 µm and D(0.9)= 39.5 µm the steps of heating under reflux, cooling and checking were carried out twice to obtain the particle sizes indicated in the following Table 4.

**Table 4**

| Example 5 | D(0.1) µm | D(0.5) µm | D(0.9) µm |
|---|---|---|---|
| Crystallized raloxifene hydrochloride (starting product) | 5.0 | 22.8 | 71.6 |
| 1st cycle (first check during the process) | 6.7 | 30.5 | 78.3 |
| 2nd cycle (second check during the process) | 9.4 | 38.5 | 93.2 |

The product leaving the second treatment cycle was then dried and granulated as in example 2 to obtain raloxifene hydrochloride with D(0.1)= 5.7µm D(0.5) = 37.4 µm and D(0.9)= 94.1 µm.

## Claims

1. A process for controlling the growth of a raloxifene hydrochloride crystal having particle size distribution D(0.9) ≤ 100µm and D(0.5) ≥ 35µm, said process comprising the steps of:
a) heating under reflux at a temperature in the range of 65 to 67°C a mass comprising crystalline raloxifene hydrochloride, methanol and water for a time of about 10 minutes,
b) cooling the mass to 30-35ºC to interrupt crystal growth,
c) checking the crystal particle size distribution,
wherein steps a), b) and c) are repeated for a number of cycles n, wherein n is from 2 to 5, until said particle size distribution is obtained and wherein once the desired crystal size has been achieved, the cooled mass is distilled under vacuum to remove part of the solvent.

2. The process according to claim 1 wherein the crystalline raloxifene hydrochloride compound of step a) is obtained by crystallization of the crude raloxifene hydrochloride in alcoholic solvent.

3. The process according to claim 2 wherein the crystallization of the crude raloxifene hydrochloride takes place from a mass comprising methanol-water in the presence of HCI.

4. The process according to claim 3 wherein the weight ratio of water to methanol in the crude raloxifene hydrochloride crystallization mass from which the crystallized raloxifene of step a) is obtained, is about 1:12.

5. The process according to any one of claims from 1 to 4 wherein the weight ratio of water to methanol in step a) is about 1:90.

6. The process according to any one of claims from 1 to 5 wherein step c) comprises sampling the reaction mass, filtration and drying of the sample followed by checking particle size distribution by a laser technique.

## Patentansprüche

1. Verfahren zum Steuern des Wachstums eines Raloxifenhydrochlorid-Kristalls mit einer Teilchengröße-Verteilung D (0,9) ≤ 100 µm und D (0,5) ≥ 35 µm, wobei das Verfahren die folgenden Schritte umfasst:
(a) Erhitzen einer Masse, die kristallines Raloxifenhydrochlorid, Methanol und Wasser umfasst, unter Rückfluss bei einer Temperatur im Bereich von 65 bis 67 °C für eine Zeit von etwa 10 Minuten;
(b) Abkühlen der Masse auf 30 bis 35 °C, um ein Kristallwachstum zu unterbrechen;
(c) Checken der Kristallteilchen-Größenverteilung;
wobei die Schritte (a), (b) und (c) für eine Zahl von Zyklen n wiederholt werden, wobei n eine Zahl von 2 bis 5 ist, bis die Teilchengröße-Verteilung erhalten wird, und worin dann, wenn einmal die Teilchengröße-Verteilung erhalten wurde, die abgekühlte Masse unter Vakuum destilliert wird, um einen Teil des Lösungsmittels zu entfernen.

2. Verfahren nach Anspruch 1, worin die kristalline Raloxifenhydrochlorid-Verbindung von Schritt (a) erhalten wird durch Kristallisation des rohen Raloxifenhydrochlorids in einem alkoholischen Lösungsmittel.

3. Verfahren nach Anspruch 2, worin die Kristallisation des rohen Raloxifenhydrochlorids stattfindet aus einer Methanol-Wasser umfassenden Masse in Gegenwart von HCl.

4. Verfahren nach Anspruch 3, worin das Gewichtsverhältnis von Wasser zu Methanol in der rohen Raloxifenhydrochlorid-Kristallisationsmasse, aus der das kristallisierte Raloxifen von Schritt (a) erhalten wird, etwa 1 : 12 ist.

5. Verfahren nach irgendeinem der Ansprüche 1 bis 4, worin das Gewichtsverhältnis von Wasser zu Methanol in Schritt (a) etwa 1 : 90 ist.

6. Verfahren nach irgendeinem der Ansprüche 1 bis 5, worin Schritt (c) ein Abnehmen einer Probe der Reaktionsmasse, ein Filtern und Trocknen der Probe, gefolgt von einem Checken der Teilchengröße-Verteilung durch eine Laser-Technik, umfasst.

## Revendications

1. Procédé de maîtrise de la croissance d'un cristal de chlorhydrate de raloxifène ayant une distribution granulométrique D(0,9) ≤ 100 µm et D(0,5) ≥ 35 µm, ledit procédé comprenant les étapes consistant à :
a) chauffer au reflux à une température dans la plage de 65 à 67 °C une masse comprenant du chlorhydrate de raloxifène cristallin, du méthanol et de l'eau pendant une durée d'environ 10 minutes,
b) refroidir la masse à 30-35 °C pour interrompre la croissance des cristaux,
c) contrôler la distribution granulométrique des cristaux,
dans lequel les étapes a), b) et c) sont répétées pendant un nombre n de cycles, où n est compris entre 2 et 5, jusqu'à ce que ladite distribution granulométrique soit obtenue et dans lequel une fois que la taille cristalline désirée a été atteinte, la masse refroidie est distillée sous vide pour enlever une partie du solvant.

2. Procédé selon la revendication 1, dans lequel le composé de chlorhydrate de raloxifène cristallin de l'étape a) est obtenu par cristallisation du chlorhydrate de raloxifène brut dans un solvant alcoolique.

3. Procédé selon la revendication 2 dans lequel la cristallisation du chlorhydrate de raloxifène brut se fait à partir d'une masse comprenant du méthanol-eau en présence de HCl.

4. Procédé selon la revendication 3 dans lequel le rapport pondéral de l'eau au méthanol dans la masse de cristallisation du chlorhydrate de raloxifène brut à partir de laquelle le raloxifène cristallisé de l'étape a) est obtenu, est d'environ 1:12.

5. Procédé selon l'une quelconque des revendications 1 à 4 dans lequel le rapport pondéral de l'eau au méthanol dans l'étape a) est d'environ 1:90.

6. Procédé selon l'une quelconque des revendications 1 à 5 dans lequel l'étape c) comprend le prélèvement d'un échantillon de la masse réactionnelle, la filtration et le séchage de l'échantillon suivis par le contrôle de la distribution granulométrique par une technique laser.
